# EUROPEAN PATENT APPLICATION

(11) **EP 4 273 548 A2**
(43) Date of publication of application: **08.11.2023**
(21) Application number: 23184303.8
(22) Date of filing: 20.02.2018
(51) Int. Cl.: G01N 33/53

(54) **SEROLOGIC ASSAY FOR SILENT BRAIN ISCHEMIA**

(30) Priority: 20.02.2017 US 201762461161 P
(62) Divisional of application: 18755090.0
(71) Applicant: The Regents of the University of California, Oakland, CA 94607 (US)
(72) Inventor: HINMAN, Jason D., Los Angeles, CA 90024 (US); XIAO, Guanxi, Los Angeles, CA 90024 (US)
(74) Representative: FRKelly

(57) **Abstract**

A method for detection or monitoring status of silent brain ischemia (SBI) and cerebrovascular health. The assay reagents and methods described herein provide a specific indicator of cerebral microvascular disease, enabling clinicians to identify patients at risk for the development of SBI. A method of treating a subject having silent brain ischemia and/or metabolic syndrome comprises administering to the subject aspirin therapy, blood pressure therapy, body weight management, and/or a program of diet and exercise when levels of two or more SBI markers are elevated. Described herein are molecules that are produced by cerebral endothelial cells exposed to chronic vascular risk factors including obesity, hyperlipidemia, hypertension, and glucose intolerance. These stress molecules produced by cerebral endothelial cells are detectable in the serum and serve as diagnostic indicators of brain-specific endothelial cell damage and correlate with MRI indicators of silent stroke and impaired cognitive function.

## Description

This application claims benefit of United States provisional patent application number 62/461,161, filed February 20, 2017, the entire contents of which are incorporated by reference into this application.

### BACKGROUND OF THE INVENTION

Obesity is a leading public health problem in the US. More than one-third of adults are obese. It is closely related to development of the metabolic syndrome, which produces various vascular risk factors, including hyperglycemia, hyperlipidemia, hypertension and low high-density lipoprotein. These factors increase the risk of developing chronic vascular diseases, particularly cerebral vascular disease. Studies show that patients with metabolic syndrome have a six-fold increase in the risk of developing microvascular infarcts in the brain, which predominantly injure brain white matter leading to dementia, disability and even death.

Millions of Americans each year have silent strokes, another name for these silent stroke are microvascular infarcts in the brain. During a silent stroke, an interruption in blood flow damages a part of the brain that does not control vital functions. Although the damage can be detected on an MRI or CT scan, it is too small to produce any obvious symptoms.

Currently, clinicians utilize brain MRI after the onset of clinical symptoms to diagnose cerebral microvascular disease. This approach is highly limited and only useful to prevent additional damage, rather than to identify patients at risk who could potentially benefit from more aggressive pharmacologic and lifestyle interventions well before lasting brain damage has set in. The current field standard for predictive assays for stroke are based on patients arriving to medical providers at the time of acute stroke rather than identifying the silent form of stroke that increases the risk of long-term consequences including disability, dementia and death. In addition, none of these predictive assays have been based on discovery based research but rather using a case-control methodology that is necessarily limited and generally uses a single molecular profile rather than an array of targets.

There are no diagnostic tests that can predict individual risk of stroke and none that can actually indicate brain blood vessel health. There remains a need for a practical and accessible diagnostic tool for identifying patients at risk of and suffering from silent stroke, facilitating timely therapeutic intervention.

### SUMMARY OF THE INVENTION

The assay reagents and methods described herein meet these needs and others by providing a specific indicator of cerebral microvascular disease. An assay as described herein would be extremely useful to practicing clinicians of many specialties to identify patients at risk for the development of silent brain ischemia (SBI). Described herein is a method of treating a subject having silent brain ischemia and/or metabolic syndrome. The method comprises administering to the subject aspirin therapy, blood pressure therapy, body weight management, and/or a program of diet and exercise when levels of two or more SBI markers are elevated.

Described herein are molecules that are produced by cerebral endothelial cells exposed to chronic vascular risk factors including obesity, hyperlipidemia, hypertension, and glucose intolerance. These stress molecules are produced by cerebral endothelial cells and detectable in the serum. These molecules serve as diagnostic indicators of brain-specific endothelial cell damage and correlate with MRI indicators of silent stroke and impaired cognitive function.

Provided hereinbelow is a list of markers for this assay. Tier 1 molecules (CXCL5/6, IGFBP2, ITGB3, IL-17B) are preferred molecules and each can serve as an independent marker of silent cerebrovascular injury. Tier 2 molecules (IL-17A, GDF-15, FGF-23, MCP-1) provide additional markers. These Tier 2 markers, as well as other markers (such as TNFa, IL-18, IL-6, Fibrinogen, BDNF, ST2, SRAGE, MPO, and LpPLA2) can be used in combination with one or more Tier 1 markers. The combination of one or more of these markers with one or more Tier 1 markers will provide additional diagnostic accuracy above each any one assay component. The panel of markers provide longitudinal predictive value and can be modulated by various pharmacologic or lifestyle interventions.

In one embodiment, the method, kit, or assay is directed at detection and/or measurement any one of IGFBP2, ITGB3, CXCL5, and/or CXCL6, or a combination thereof, in a sample obtained from a subject. In one embodiment, the marker to be measured is CXCL5. In one embodiment, the marker to be measured is CXCL6. In another embodiment, the marker to be measured is IGFBP2. In one embodiment, the marker to be measured is ITGB3. Typically, at least two or three markers are measured. In certain embodiments, CXCL5 and IGFBP2 are both measured. In some embodiments, CXCL5, CXCL6, and IGFBP2 are measured. In other embodiments, CXCL5, IGFBP2, and ITGB3 are measured.

In one embodiment, the marker is at least one marker selected from Table 1 or Table 2. In one embodiment, at least two or more markers of Table 1, 2 and/or 3 is used in combination. Examples of combinations of markers include: CXCL5 and an additional marker selected from Table 1, 2, or 3; CXCL6 and an additional marker selected from Table 1, 2, or 3; IGFBP2 and an additional marker selected from Table 1, 2, or 3; ITGB3 and an additional marker selected from Table 1, 2, or 3; IL-17B and an additional marker selected from Table 1, 2, or 3; IL-17A and an additional marker selected from Table 1, 2, or 3; GDF-15 and an additional marker selected from Table 1, 2, or 3; FGF-23 and an additional marker selected from Table 1, 2, or 3; CCL2/MCP-1 and an additional marker selected from Table 1, 2, or 3; IL-6 and an additional marker selected from Table 1, 2, or 3; TNF-alpha and an additional marker selected from Table 1, 2, or 3; CXCL5 and CXCL6 and an additional marker selected from Table 1, 2, or 3; any combination of 3 or more markers selected from Tables 1, 2 and 3; any combination of 4, 5, 6, 7, 8, 9, 10, or 11 markers of Tables 1, 2 and 3. In one embodiment, the combination of markers is 2, 3, 4, or 5 markers selected from Table 1. In another embodiment, the combination of markers is at least one marker from Table 1, and at least one marker from Table 2. In another embodiment, the combination of markers is at least one marker from Table 1, and at least one marker from Table 3. In another embodiment, the combination of markers is at least one marker from Table 2, and at least one marker from Table 3. In another embodiment, the combination of markers is at least one marker from each of Tables 1, 2, and 3.

Table 4 above lists markers that are both novel and previously reported, as indicated. In some embodiments, a combination of two or more of the markers listed in Table 4 is used in a method or assay as described herein. In some embodiments, 3, 4, 5, 6, 7, 8, 9, 10, 11, 12, 13, 14, 15, or all 16 of the markers listed in Table 4 are used together in an assay. Optionally, all or a subset of these markers can be used in conjunction with other markers described herein or elsewhere.

The invention provides methods for monitoring cerebrovascular status in a sample obtained from a subject, for diagnosing silent stroke in a subject, as well as methods for predicting, treating, and/or monitoring silent stroke, cerebrovascular injury, and/or metabolic syndrome.

In a typical embodiment, the method comprises:
(a) contacting a sample obtained from the subject with reagents that specifically bind to at least one marker selected from Table 1 or Table 2;
(b) measuring the level of binding to the marker;
(c) assigning a status score that reflects the measured amount of marker relative to a normal control;
(d) referring the subject for treatment of metabolic syndrome and/or stroke if the status score is significantly greater than 1.

In some embodiments, the method further comprises treating the subject for metabolic syndrome and/or stroke. Examples of treatment include, but are not limited to, aspirin therapy, blood pressure therapy, body weight management, and/or a program of diet and exercise.

Also provided is a method of monitoring silent stroke in a subject. In one embodiment, the method comprises:
(a) contacting a sample obtained from the subject with reagents that specifically bind to at least one marker selected from Table 1 or Table 2;
(b) measuring the level of binding to the marker;
(c) assigning a status score that reflects the measured amount of marker relative to a normal control;
(d) treating the subject for metabolic syndrome and/or stroke if the status score is significantly greater than 1;
(e) repeating steps (a) to (c); and
(f) adjusting the treatment when the status score is not trending toward 1.

Also provided is a method of treating silent stroke and/or metabolic syndrome in a subject in need thereof. In one embodiment, the method comprises:
(a) measuring a level of at least one marker selected from Table 1;
(b) administering to the subject aspirin therapy, blood pressure therapy, body weight management, and/or a program of diet and exercise when the level of the marker is elevated.

In some embodiments, the at least one marker comprises C-X-C motif chemokine 5 (CXCL5) and/or C-X-C motif chemokine 6 (CXCL6). In some embodiments, the at least one marker comprises IGFBP2. In some embodiments, the measuring of step (a) is performed for at least two of markers of Table 1. In other embodiments, the measuring of step (a) is performed for at least three of the markers of Table 1 and/or Table 2. In some embodiments, the method further comprises measuring a marker of Table 3.

In typical embodiments, the sample is a serum sample, CSF sample, a urine sample, a blood sample, or other bodily fluid. For use in the methods described herein, representative examples of the sample include, but are not limited to, blood, plasma or serum, saliva, urine, cerebral spinal fluid, milk, cervical secretions, semen, and other bodily fluids.

The subject is typically a mammalian subject, such as a human. In some embodiments, the subject is a veterinary subject, such as a pet or other companion animal.

In some embodiments, the reagents are antibodies. In other embodiments, the reagents are nucleic acid probes capable of specifically hybridizing with a target for detection. The reagents can optionally be labeled with a detectable marker. The methods can be performed using, for example, immunoassay techniques, such as enzyme immunoassays, multiplex assays. Other assays can be employed, such as probe hybridization, as will be understood to those skilled in the art.

The invention provides kits comprising a set of reagents as described herein, such as antibodies that specifically bind one or more markers of the invention, and optionally, one or more suitable containers containing reagents of the invention. Reagents include molecules that specifically bind one or more markers of the invention. One example of a reagent is an antibody that is specific for the marker. Reagents can optionally include a detectable label. Labels can be fluorescent, luminescent, enzymatic, chromogenic, or radioactive.

Kits of the invention optionally comprise an assay standard or a set of assay standards, either separately or together with other reagents. An assay standard can serve as a normal control by providing a reference level of normal expression for a given marker that is representative of a healthy individual.

Kits can include probes for detection of alternative gene expression products in addition to antibodies for protein detection. The kit can optionally include a buffer.

In one embodiment, the kit comprises antibodies or probes that specifically bind to expression products of CXCL5, CXCL6, IGFBP2, and/or ITGB3. In one embodiment,the kit comprises reagents that specifically bind CXCL5, CXCL6, IGFBP2, and ITGB3. In some embodiments, the kit comprises reagents that specifically bind CXCL5 and IGFBP2. Such kits can optionally further comprise reagents that specifically bind CXCL6 and/or ITGB3. Typically, the kit comprises antibodies or probes that specifically bind to at least two of CXCL5, CXCL6, IGFBP2, and/or ITGB3. In some embodiments, the antibodies or probes of the kit specifically at least three, or optionally, all four, of CXCL5, CXCL6, IGFBP2, and/or ITGB3. Optionally, the kit can further comprise antibodies or probes that specifically bind to 1, 2, 3, 4, 5, 6, 7, 8, 9, or up to 10 additional expression products. The additional expression products can be selected from Tables 2, 3, or 4 herein. Optionally, the additional expression products can be other markers of interest.

### BRIEF DESCRIPTION OF THE FIGURES

Figure 1 is a bar graph showing weight-adjusted serum levels of CXCL5, confirming that one of the up-regulated diseased endothelial markers was secreted in the mouse and is up-regulated in retro-orbital blood draining from the brain in mice with metabolic syndrome.
Figure 2 is a scatterplot showing the CXCL5 and CXCL6 data.
Figure 3 is a scatterplot showing that significant correlations were found between serum levels of CXCL5 and CXCL6 and Trails A performance.
Figure 4 is a bar graph showing that significant differneces were found between subjects having CXCL5 serum levels below 500 pg/mL versus above 500 pg/mL in Trails A performance.
Figure 5 is a bar graph showing that significant differneces were found between subjects having CXCL5 serum levels below 500 pg/mL versus above 500 pg/mL in Digit Span performance.
Figure 6 is a bar graph showing Fazekas scores, a measure of white matter lesions.
Figure 7 shows the results of technical validation, analyzing percent coefficient of variation (CoV) observed between plates in healthy controls.
Figure 8 shows the results of technical validation, analyzing percent coefficient of variation (CoV) observed between subjects.
Figure 9 is a scatterplot that shows serum myeloperoxidase (MPO) levels as a function of Fazekas scores (FS), which weakly correlated.
Figure 10 is a scatterplot that shows that IGFPB2 serum levels were significantly correlated with FS scores.

### DETAILED DESCRIPTION

The invention is based on the unexpected discovery of molecules that are produced by cerebral endothelial cells exposed to chronic vascular risk factors, including obesity, hyperlipidemia, hypertension, and glucose intolerance. These stress molecules are produced by cerebral endothelial cells and detectable in the serum. The invention provides an assay using these molecules as diagnostic indicators of brain-specific endothelial cell damage, and which correlates with MRI indicators of silent stroke and impaired cognitive function. The assay reagents and methods described herein provide a specific indicator of cerebral microvascular disease. These indicators are extremely useful to practicing clinicians of many specialties, allowing them to identify patients at risk for the development of silent brain ischemia (SBI), which can otherwise go undetected. The markers can also be used as a non-invasive screening tool for the detection and treatment of small vessel ischemic disease (SVID) and other forms of cerebrovascular disease (CVD).

### Definitions

All scientific and technical terms used in this application have meanings commonly used in the art unless otherwise specified. As used in this application, the following words or phrases have the meanings specified.

As used herein, a "control" sample means a sample that is representative of normal measures of the respective marker, such as would be obtained from normal, healthy control subjects, or a baseline amount of marker to be used for comparison. Typically, a baseline will be a measurement taken from the same subject or patient. The sample can be an actual sample used for testing, or a reference level or range, based on known normal measurements of the corresponding marker.

As used herein, "The term "nucleic acid" or "polynucleotide" or "oligonucleotide" refers to a sequence of nucleotides, a deoxyribonucleotide or ribonucleotide polymer in either single- or double-stranded form, and unless otherwise limited, encompasses known analogs of natural nucleotides that hybridize to nucleic acids in a manner similar to naturally occurring nucleotides.

As used herein, "hybridizes," "hybridizing," and "hybridization" means that the oligonucleotide forms a noncovalent interaction with the target DNA molecule under standard conditions. Standard hybridizing conditions are those conditions that allow an oligonucleotide probe or primer to hybridize to a target DNA molecule. Such conditions are readily determined for an oligonucleotide probe or primer and the target DNA molecule using techniques well known to those skilled in the art. The nucleotide sequence of a target polynucleotide is generally a sequence complementary to the oligonucleotide primer or probe. The hybridizing oligonucleotide may contain non-hybridizing nucleotides that do not interfere with forming the noncovalent interaction. The nonhybridizing nucleotides of an oligonucleotide primer or probe may be located at an end of the hybridizing oligonucleotide or within the hybridizing oligonucleotide. Thus, an oligonucleotide probe or primer does not have to be complementary to all the nucleotides of the target sequence as long as there is hybridization under standard hybridization conditions.

As used herein, a "significant difference" means a difference that can be detected in a manner that is considered reliable by one skilled in the art, such as a statistically significant difference, or a difference that is of sufficient magnitude that, under the circumstances, can be detected with a reasonable level of reliability. As used herein, "a" or "an" means at least one, unless clearly indicated otherwise.

As used herein, to "prevent" or "protect against" a condition or disease means to hinder, reduce or delay the onset or progression of the condition or disease.

As used herein, "a" or "an" means at least one, unless clearly indicated otherwise.

### Markers

Described herein are molecules that are produced by cerebral endothelial cells exposed to chronic vascular risk factors including obesity, hyperlipidemia, hypertension, and glucose intolerance. These stress molecules are produced by cerebral endothelial cells and detectable in the serum. These molecules serve as diagnostic indicators of brain-specific endothelial cell damage and correlate with MRI indicators of silent stroke and impaired cognitive function.

Provided below is a list of markers for this assay. Tier 1 molecules (Table 1) are our strongest candidate molecules and each can serve as an independent marker of silent cerebrovascular injury. Tier 2 molecules (Table 2) provide additional markers. The combination of these markers will provide additional diagnostic accuracy above each any one assay component. The panel of markers provide longitudinal predictive value and can be modulated by various pharmacologic or lifestyle interventions. Previously known markers of cardiovascular disease (CVD), such as those listed in Table 3, can be used in conjunction with one or more markers listed in Tables 1 and 2.

**Table 1**

| Protein Acronym | Full Name |
|---|---|
| CXCL5 (ENA-78) | C-X-C motif chemokine 5 |
| CXCL6 (GCP-2) | C-X-C motif chemokine 6 |
| IGFBP2 | Insulin-like growth factor-binding protein 2 |
| ITGB3 (CD61) | Integrin, beta 3 (platelet glycoprotein IIIa) |
| IL-17B | lnterleukin-17-B |

**Table 2**

| Protein Acronym | Full Name |
|---|---|
| IL-17A | Interleukin-17-A |
| GDF-15 | Growth/differentiation factor 15 |
| FGF-23 | Fibroblast growth factor-23 |
| CCL2/MCP-1 | Chemokine (C-C motif) ligand 2 |

**Table 3**

| Protein Acronym | Full Name |
|---|---|
| IL-6 | Interleukin-6 |
| TNF-alpha | Tumor necrosis factor-alpha |

In one embodiment, the marker is at least one marker selected from Table 1 or Table 2. In one embodiment, at least two or more markers of Table 1, 2 and/or 3 is used in combination. Examples of combinations of markers include: CXCL5 and an additional marker selected from Table 1, 2, or 3; CXCL6 and an additional marker selected from Table 1, 2, or 3; IGFBP2 and an additional marker selected from Table 1, 2, or 3; ITGB3 and an additional marker selected from Table 1, 2, or 3; IL-17B and an additional marker selected from Table 1, 2, or 3; IL-17A and an additional marker selected from Table 1, 2, or 3; GDF-15 and an additional marker selected from Table 1, 2, or 3; FGF-23 and an additional marker selected from Table 1, 2, or 3; CCL2/MCP-1 and an additional marker selected from Table 1, 2, or 3; IL-6 and an additional marker selected from Table 1, 2, or 3; TNF-alpha and an additional marker selected from Table 1, 2, or 3; CXCL5 and CXCL6 and an additional marker selected from Table 1, 2, or 3; any combination of 3 or more markers selected from Tables 1, 2 and 3; any combination of 4, 5, 6, 7, 8, 9, 10, or 11 markers of Tables 1, 2 and 3. In one embodiment, the combination of markers is 2, 3, 4, or 5 markers selected from Table 1. In another embodiment, the combination of markers is at least one marker from Table 1, and at least one marker from Table 2. In another embodiment, the combination of markers is at least one marker from Table 1, and at least one marker from Table 3. In another embodiment, the combination of markers is at least one marker from Table 2, and at least one marker from Table 3. In another embodiment, the combination of markers is at least one marker from each of Tables 1, 2, and 3.

C-X-C motif chemokine ligand 5 (CXCL5) is a member of the CXC subfamily of chemokines. CXCL5 is also known as small inducible cytokine subfamily B (Cys-X-Cys), member 5 (SCYB5) and ENA-78. Chemokines, which recruit and activate leukocytes, are classified by function (inflammatory or homeostatic) or by structure. This protein is proposed to bind the G-protein coupled receptor chemokine (C-X-C motif) receptor 2 to recruit neutrophils, to promote angiogenesis and to remodel connective tissues. This protein is thought to play a role in cancer cell proliferation, migration, and invasion.

C-X-C motif chemokine ligand 6 (CXCL6) is another member of the CXC subfamily of chemokines. CXCL6 is also known as granulocyte chemotactic protein 2 (GCP2), CKA-3, and SCYB6. In addition, to being chemotactic for neutrophil granulocytes (signaling through binding and activation of its receptors, CXCR1 and CXCR2), and angiogenic, it has strong antibacterial activity against Gram-positive and Gram-negative bacteria (90-fold-higher when compared to CXCL5 and CXCL7).

Insulin-like growth factor-binding protein 2 (IGFBP2) inhibits IGF-mediated growth and developmental rates. IGF-binding proteins prolong the half-life of the IGFs and have been shown to either inhibit or stimulate the growth promoting effects of the IGFs on cell culture. They alter the interaction of IGFs with their cell surface receptors.

The Integrin beta-3 (ITGB3) protein product is the integrin beta chain beta 3. Integrins are integral cell-surface proteins composed of an alpha chain and a beta chain. A given chain may combine with multiple partners resulting in different integrins. Integrin beta 3 is found along with the alpha IIb chain in platelets. Integrins are known to participate in cell adhesion as well as cell-surface mediated signaling.

In one embodiment, the method, kit, or assay is directed at detection and/or measurement of IGFBP2, ITBG3, CXCL5, and/or CXCL6 in a sample obtained from a subject.

**Table 4**

| Analyte | Novel/Reported |
|---|---|
| IGFBP2 | Novel |
| ITBG3 | Novel |
| CXCL5 | Novel |
| CXCL6 | Novel |
| TN Fa | Jefferson et al. Neurology 2007 |
| IL-18 | Miwa et al. Stroke 2011 |
| IL-6 | Nadkami et al. Neurology 2016 |
| Fibrinogen | Aono et al. Arterioscler Thromb Vasc Biol 2007 |
| MCP-1 | Bettcher et al. Alzheimers Dement 2016 |
| BDNF | Pikula et al. Stroke 2013 |
| GDF-15 | Andersson et al. Stroke 2015 |
| ST2 | Andersson et al. Stroke 2015 |
| FGF-23 | Wright et al. Stroke 2016 |
| SRAGE | Hudson et al. Atherosclerosis 2011 |
| MPO | Shoamanesh et al. Neurology 2015 |
| LpPLA2 | Shoamanesh et al. Neurology 2015 |

Table 4 above lists markers that are both novel and previously reported, as indicated. In some embodiments, a combination of two or more of the markers listed in Table 4 is used in a method or assay as described herein. In some embodiments, 3, 4, 5, 6, 7, 8, 9, 10, 11, 12, 13, 14, 15, or all 16 of the markers listed in Table 4 are used together in an assay. Optionally, all or a subset of these markers can be used in conjunction with other markers described herein or elsewhere.

### Methods of the Invention

Described herein is a method of treating a subject having silent brain ischemia and/or metabolic syndrome. The method comprises administering to the subject aspirin therapy, blood pressure therapy, blood sugar control, cholesterol management, body weight management, and/or a program of diet and exercise when levels of two or more SBI markers are elevated.

The invention provides methods for monitoring cerebrovascular status in a sample obtained from a subject, for diagnosing silent stroke in a subject, as well as methods for predicting, treating, and/or monitoring silent stroke, cerebrovascular injury, cognitive impairment, and/or metabolic syndrome.

In a typical embodiment, the method comprises:
(a) contacting a sample obtained from the subject with reagents that specifically bind to at least one marker selected from Table 1 or Table 2;
(b) measuring the level of binding to the marker;
(c) assigning a status score that reflects the measured amount of marker relative to a normal control;
(d) referring the subject for treatment of metabolic syndrome and/or stroke if the status score is significantly greater than 1.

In some embodiments, the method further comprises treating the subject for metabolic syndrome and/or stroke. Examples of treatment include, but are not limited to, aspirin therapy, blood pressure therapy, body weight management, and/or a program of diet and exercise.

Also provided is a method of monitoring silent stroke in a subject. In one embodiment, the method comprises:
(a) contacting a sample obtained from the subject with reagents that specifically bind to at least one marker selected from Table 1 or Table 2;
(b) measuring the level of binding to the marker;
(c) assigning a status score that reflects the measured amount of marker relative to a normal control;
(d) treating the subject for metabolic syndrome and/or stroke if the status score is significantly greater than 1;
(e) repeating steps (a) to (c); and
(f) adjusting the treatment when the status score is not trending toward 1.

Also provided is a method of treating silent stroke and/or metabolic syndrome in a subject in need thereof. In one embodiment, the method comprises:
(a) measuring a level of at least one marker selected from Table 1;
(b) administering to the subject aspirin therapy, blood pressure therapy, body weight management, and/or a program of diet and exercise when the level of the marker is elevated.

In some embodiments, the at least one marker comprises C-X-C motif chemokine 5 (CXCL5) and/or C-X-C motif chemokine 6 (CXCL6). In some embodiments, the at least one marker comprises IGFBP2. In some embodiments, the measuring of step (a) is performed for at least two of markers of Table 1. In other embodiments, the measuring of step (a) is performed for at least three of the markers of Table 1 and/or Table 2. In some embodiments, the method further comprises measuring a marker of Table 3.

In typical embodiments, the sample is a CSF sample, a urine sample, a blood sample, or other bodily fluid. For use in the methods described herein, representative examples of the sample include, but are not limited to, blood, plasma or serum, saliva, urine, cerebral spinal fluid, milk, cervical secretions, semen, and other bodily fluids.

The subject is typically a mammalian subject, such as a human. In some embodiments, the subject is a veterinary subject, such as a pet or other companion animal.

The methods can be performed using, for example, immunoassay techniques, such as enzyme immunoassays. Other assays can be employed, as will be understood to those skilled in the art.

### Kits and Assay Standards

The invention provides kits comprising a set of reagents as described herein, such as antibodies that specifically bind one or more markers of the invention, and optionally, one or more suitable containers containing reagents of the invention. Reagents include molecules that specifically bind one or more markers of the invention. One example of a reagent is an antibody that is specific for the marker. Reagents can optionally include a detectable label. Labels can be fluorescent, luminescent, enzymatic, chromogenic, or radioactive.

Kits of the invention optionally comprise an assay standard or a set of assay standards, either separately or together with other reagents. An assay standard can serve as a normal control by providing a reference level of normal expression for a given marker that is representative of a healthy individual.

Kits can include probes for detection of alternative gene expression products in addition to antibodies for protein detection. The kit can optionally include a buffer.

In one embodiment, the kit comprises antibodies or probes that specifically bind to expression products of CXCL5, CXCL6, IGFBP2, and/or ITGB3. Typically, the kit comprises antibodies or probes that specifically bind to at least two of CXCL5, CXCL6, IGFBP2, and/or ITGB3. In some embodiments, the antibodies or probes of the kit specifically at least three, or optionally, all four, of CXCL5, CXCL6, IGFBP2, and/or ITGB3. Optionally, the kit can further comprise antibodies or probes that specifically bind to 1, 2, 3, 4, 5, 6, 7, 8, 9, or up to 10 additional expression products. The additional expression products can be selected from Tables 2, 3, or 4 herein. Optionally, the additional expression products can be other markers of interest.

### EXAMPLES

The following examples are presented to illustrate the present invention and to assist one of ordinary skill in making and using the same. The examples are not intended in any way to otherwise limit the scope of the invention.

### Example 1: Dramatic alteration of microvascular complexity and endothelial cell gene expression in mice with metabolic syndrome

This Example describes the effects of metabolic syndrome on cerebral microvasculature and unveils the molecular mechanisms that regulate these changes. This syndrome in mice strongly mimics the human condition marked by obesity, elevated cholesterol, and impaired glucose tolerance. This constellation of symptoms greatly increase the risk of stroke (6-fold), particularly silent stroke. Silent stroke damages brain white matter and leads to disability, dementia and death.

### Methods

Transgenic mice (Tie2-Cre:flox-stop tdtomato plus flox-RiboTAG) were fed with a 60%kCal from fat diet to induce metabolic syndrome. Subjects were fed the high fat diet for 12 weeks at 2 months age, and a 10%kCal from fat diet was used as control. The volume of cerebral vasculature in white matter was evaluated by reporter gene expression in endothelial cells of Tie2-Cre:flox-stop tdtomato mice. Transcriptomes from cerebral endothelial cells were isolated using Ribotag ribosomal immunoprecipitation technology (Tie2-Cre:flox-RiboTAG). Following ribosomal associated transcriptome isolation, RNA-seq was performed.

### Results

Diet-induced obesity creates a metabolic syndrome serologic profile, as shown in the following table.

| Diet | Total Cholesterol | HDL | [0074] Triglycerides | LDL | Glucose | HgbA1c |
|---|---|---|---|---|---|---|
| Control (n=3) | 110.7 | 56.0 | 88.7 | 37.0 | 162.0 | 5.6 |
| High Fat | 187.3* | 68.3* | 96.0 | 99.7* | 373.3* | 6.6 |
| (n=3) | *p<0.007 | | | | | |

Metabolic syndrome results in a significant decrease in white matter vascular volume in both large (30±2.2%, P< 0.04) and micro vessel (23±3.7%, P<0.01). Transcriptional profiling of white matter endothelial cells reveal a difference of expression profile between normal mice and mice with metabolic syndrome. The significant changes in endothelial cell gene expression (see Example 2) are associated with the decreased volume of vessels in white matter with metabolic syndrome through changes in angiogenic receptor ligands.

Metabolic syndrome decreases the volumes of large and micro vessels in white matter and significantly changes endothelial cell gene expression. These findings help to develop molecular therapeutic approaches for patients with metabolic syndrome to prevent cerebral microvascular complications including stroke.

### Example 2: Immunoassay for markers of brain endothelial blood vessel damage

This Example uses the mouse model of obesity that creates 'metabolic syndrome' described in Example 1. Using a new transgenic mouse technology with this mouse model of obesity, we have identified a series of molecular indicators of early cerebral blood vessel damage that occur in the setting of obesity and these common metabolic disturbances. A number of these genes code for proteins that are secreted in the blood and can therefore be used to indicate early brain blood damage

This has led to the development of a multispot enzyme immunoassay (EIA) test for the secreted molecules in the endothelial data set described herein that mark brain endothelial blood vessel damage.

### Methods

Samples of a patient's plasma are serially diluted and pipetted into individual wells of a microtiter plate. Novel antibodies created against 5-10 of our unique obesity-induced cerebral endothelial genes are primarily labeled with fluorescent indicators. Up to 4 different antibodies with non-overlapping fluorescent indicators are incubated with each patient sample well. The plate is repeatedly washed to remove non-specific binding, and then scanned in a multichannel fluorescent spectrophotometer, and the fluorescent intensity of each channel for each well is recorded. This approach is repeated until all molecules in the profile have been measured. The cumulative fluorescent intensity is averaged across all targets and compared to a normative value.

Below is a list of markers that can be used in this assay, including some previously identified molecules that serve as internal controls. Table 1 molecules are preferred molecules, and each can serve as an independent marker (e.g., used alone) of silent cerebrovascular injury. CXCL5 and CXCL6 have demonstrated independent predictive ability in experimental studies. Table 2 molecules include additional markers associated with diseased endothelia and cerebrovascular injury that can be detected in serum for use as an indicator of silent stroke as described herein. Table 3 molecules include additional known markers associated with diseased endothelia and cerebrovascular injury that can be detected in serum for use as an indicator of silent stroke as described herein. The use of a combination of these markers will provide additional diagnostic accuracy above use of a single assay reagent. The panel of markers can provide longitudinal predictive value and be modulated by various pharmacologic or lifestyle interventions.

**Table 1**

| Protein Acronym | Full Name |
|---|---|
| CXCL5 (ENA-78) | C-X-C motif chemokine 5 |
| CXCL6 (GCP-2) | C-X-C motif chemokine 6 |
| IGFBP2 | Insulin-like growth factor-binding protein 2 |
| ITGB3 (CD61) | Integrin, beta 3 (platelet glycoprotein IIIa) |
| IL-17B | lnterleukin-17-B |

**Table 2**

| Protein Acronym | Full Name |
|---|---|
| IL-17A | Interleukin-17-A |
| GDF-15 | Growth/differentiation factor 15 |
| FGF-23 | Fibroblast growth factor-23 |
| CCL2/MCP-1 | Chemokine (C-C motif) ligand 2 |

**Table 3**

| Protein Acronym | Full Name |
|---|---|
| IL-6 | Interleukin-6 |
| TNF-alpha | Tumor necrosis factor-alpha |

### Example 3: Developing for markers of brain endothelial blood vessel damage

This Example describes the studies leading to identification of novel cerebral endothelial biomarkers of small vessel cerebrovascular disease (SVD). Obesity, hypertension, hyperlipidemia, and diabetes all increase the prevalence of white matter damage and synergistically contribute to altered signaling within the cerebral microvasculature. Yet, the precise molecular pathways activated in the cerebral microvasculature by these chronic conditions remain unknown. To address this knowledge gap, a novel translational approach was utilized to identify the cell-specific transcriptional profile of cerebral endothelia within the white matter in a mouse model of obesity/metabolic syndrome. Using RiboTAG transgenic technology, in which a major ribosomal protein is genetically modified with the hemagglutinin antigen, in combination with cell-specific Cre-loxP transgenic modeling, the transcriptional profile of cerebral endothelia can be isolated. This unbiased approach identifies the first direct and isolated cerebrovascular signature of chronic vascular conditions such as obesity, glucose intolerance, and hyperlipidemia, all of which are directly associated with SVD. Using this modeling approach to rationally identify new SVD biomarkers, the Example identifies the obesity-induced cerebrovascular signature that includes cell surface and secreted molecules that may be detected in the serum.

Pathway analysis of the major genes up-regulated within the diseased cerebral endothelia reveals direct links to thrombosis (ITGB3), Alzheimer's disease (IGFBP2) and inflammatory signaling (CXCL5), thereby directly linking chronic vascular risk with Alzheimer's and vascular dementia. The list of up-regulated genes (having a sufficiently low false discovery rate, or FDR<0.1) was culled to identify those that were likely to be secreted or cell surface molecules that shed into the blood stream (see Table 1). Table 5 lists the up-regulated genes as detected by mRNA levels. LogFC refers to a log transformation of fold change difference in mRNA detection. From this list, genes likely to be secreted and that had previously been detected in human serum were selected.

**Table 5**

| Gene | Name | LogFC |
|---|---|---|
| Itgb3 | Integrin beta 3 | 10.65 |
| Ttc21a | Tetratricopeptide repeat domain 21a | 13.06 |
| Foxm1 | Forkhead box M1 | 11.62 |
| Odf2l | Outer dense fiber of sperm tails 2-like | 12.00 |
| Cxcl5 | Chemokine ligand 5 | 11.67 |
| Igfbp2 | Insulin-like growth factor binding protein 2 | 6.96 |
| Gnpda2 | Glucosamine-6-phosphate deaminase 2 | 6.87 |

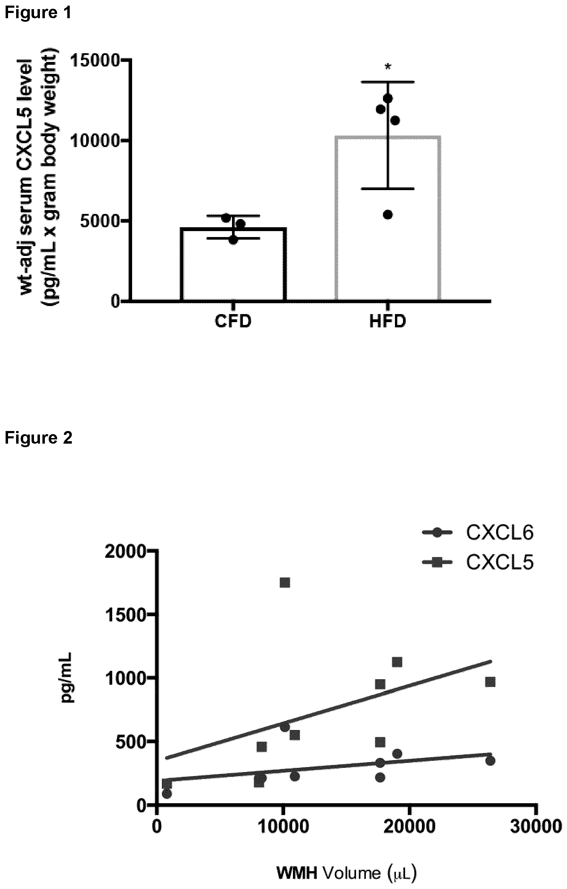

We confirmed that one of the up-regulated diseased endothelial markers was secreted in the mouse and is up-regulated in retro-orbital blood draining from the brain in mice with metabolic syndrome (Figure 1). Thus, these data demonstrate that the cerebral endothelia can produce a disease signature that can be measured in the blood stream.

### Example 4: Detecting markers of brain endothelial blood vessel damage in humans

To confirm the relevance of this cerebral endothelial molecular signature, we determined the levels of CXCL5 and CXCL6 (human ortholog to mouse CXCL5) in 15 cognitively normal subjects from the UCSF MAC (Memory and Aging Center) with varying degrees of white matter disease as indexed by volume of white matter hypointensities using Freesurfer. In subjects with at least mild SVD, the correlation between white matter hypointensity volume and our signaling factors approached significance, even in our small sample (Spearman rho for CXCL5=.56; CXCL6=.57; p's < .10). A scatterplot showing the CXCL5 and CXCL6 data is shown in Figure 2. These data demonstrate the translational validity of using these recently discovered cerebral endothelial molecules in the mouse as human biomarkers reflective of cerebral microvascular disease at the endothelial cell level.

CXCL5 and CXCL6 are two of the ELR family of chemokine ligands that bind to CXC receptor 2. These molecules are known to have a role in neutrophil homing to sites of injury and are, therefore, up-regulated by cells at the site of injury. In recent years, both molecules have been linked to the pathogenesis and progression of both atherosclerosis and SVD risk factors, though the exact mechanism is unknown and likely to involve an immunomodulatory role rather than the regulation of neutrophil migration. The CXC family of chemokines, in particular CXCL5, has been shown to have proangiogenic properties and thus may provide a unique cerebral SVD signal for angiogenesis.

Integrin alpha-V/beta-3 (ITGB3) is a multifunctional endothelial receptor whose primary function is to bind soluble fibrinogen and thereby activates platelets resulting in thrombosis. Increases in fibrinogen levels have already been established as linked to white matter lesions and vascular dementia. However, as serum fibrinogen levels can be modified by a wide variety of stimuli and the previously identified ORs are low (<2), the identification of cerebral endothelial ITGB3 as a potential ligand for fibrinogen will likely result in a biomarker with greater sensitivity than serum fibrinogen levels.

Insulin-like growth factor binding protein-2 (IGF-BP2) is a peptide hormone that is complexed to other proteins in serum and function to modulate the half-life of insulin-like growth factors (IGFs). IGF-BP2 may also have pro-angiogenic properties. IGF-BP2 has previously been linked with cortical atrophy in Alzheimer's disease and age-specific cognitive decline. Its relationship to white matter lesions has not been studied.

We recently performed longitudinal follow-up over nearly 12 years of 3374 stroke- and dementia-free individuals from the Framingham Heart study to assess the influence of GDF-15 serum levels on incident stroke and SVD. After adjustment for traditional cardiovascular risk factors such as B-type natriuretic peptide, high-sensitivity C-reactive protein, and urine albumin levels, higher GDF-15 levels were cross-sectionally associated with worse performance on a visual memory test (βs for Q4 vs. Q1=-0.62 for GDF-15, p=0.009) and greater log-transformed white-matter hyperintensity volumes (β for Q4 vs. Q1=0.19, p=0.01). This evidence suggests that GDF-15 may be an excellent marker for incipient SVD, but longitudinal studies to test this hypothesis have not been performed.

Circulating inflammatory factors: Prior work from our lab and others offers evidence that well-studied cytokines like C-reactive protein (CRP) and interleukin-6 (il-6) are linked to SVD.

We are now investigating cytokines and chemokines that could serve as more direct markers of endothelial function. Monocyte chemoattractant protein (CCL-2; MCP-1), for example, may play a critical role in the development of vascular disease by causing diapedesis of monocytes from the lumen to the subendothelial space where they become foam cells, initiating fatty streak formation that leads to atherosclerotic plaque formation. We studied 131 functionally intact older subjects (mean age=72.7) who had longitudinal 3T structural MRI. MCP-1 was measured in plasma using the Mesoscale vplex chemokine panel. We used total volume of the corpus callosum as a general marker of white matter integrity, and our primary dependent variable was annualized rate of change in corpus callosum volume. Using multiple regression controlling for age and intracranial volume, we found that higher levels of MCP-1 were associated with a steeper decline in corpus callosum volumes over time. These data support the possible role of endothelial-specific inflammatory molecules in cerebrovascular health.

We have identified several important proteomic markers of cerebral endothelial dysfunction that can improve sensitivity to early SVD and serve as predictors of SVD progression.

### Example 5: Association of markers with cognitive impairment and small vessel ischemic disease

This Example supports the association of markers listed in Table 1 with cognitive impairment, and also supports the association of the marker IGFBP2 with imaging evidence of silent stroke. These early injury serum indicators correlate with imaging indicators of cerebral small vessel disease (CSVD), also referred to as small vessel ischemic disease (SVID).

Legacy serum samples were obtained from a prior study, selecting a small subcohort of 65 subjects having a relatively low burden of vascular disease. Subjects were cognitively phenotyped using Trails A performance on the Trail Making Test, a neuropsychological test of visual attention, and Digit Span performance, a short term memory assessment tool.

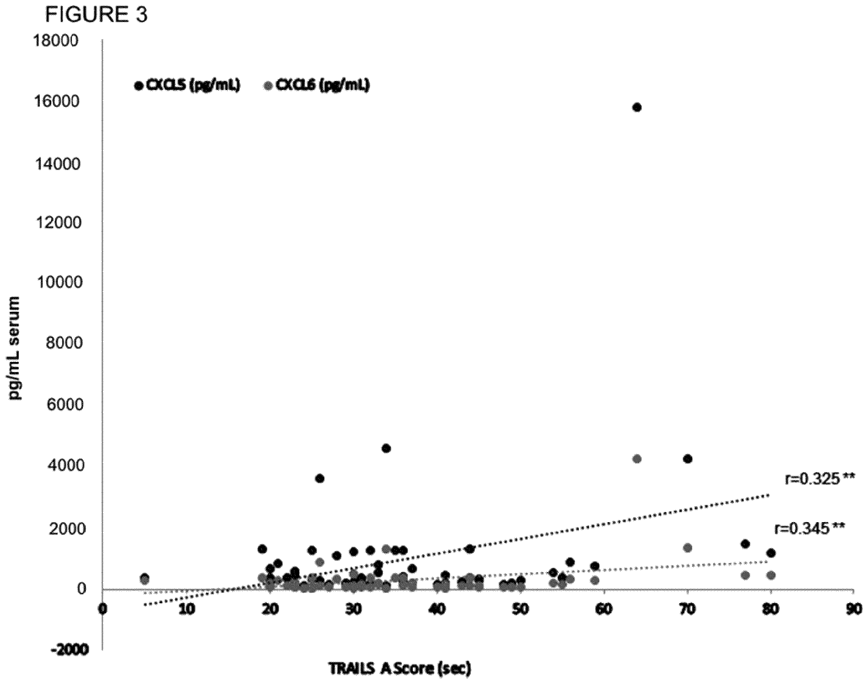

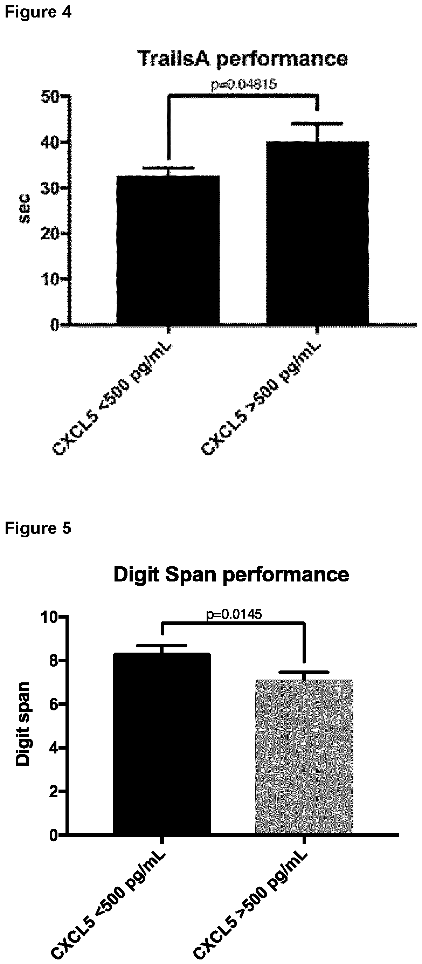

Significant correlations were found between serum levels of CXCL5 and CXCL6 and Trails A performance (Figure 3). Significant differneces were also found between subjects having CXCL5 serum levels below 500 pg/mL versus above 500 pg/mL in both Trails A performance (Figure 4) and Digit Span performance (Figure 5).

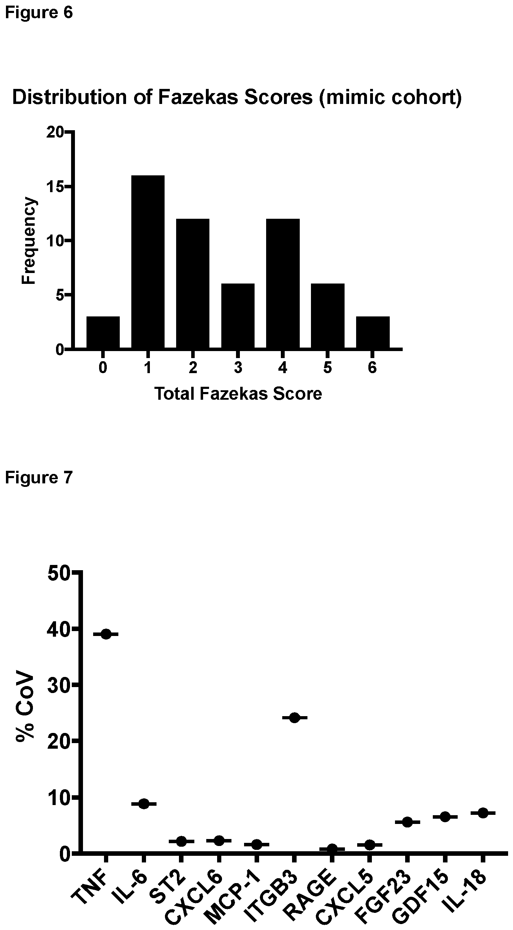

A study of recent stroke patients included consenting patients over the age of 18 who presented to an Emergency Department over a span of six months with acute neurological symptoms (within previous 24 hours). White matter hyperintensities were measured on axial T2-weighted fluid-attenuated inversion recovery (FLAIR) images using modified Fazekas scoring. Out of 202 subjects initially enrolled, only 168 had an MRI taken. Of these (MRI taken), 100 were negative for diffusion weighted imaging, which detects acute ischemic stroke. Of these 100 subjects, 42 showed evidence on imaging of stroke/TIA, while 58 did not show evidence of stroke. Figure 6 is a bar graph showing Fazekas scores, a measure of white matter lesions.

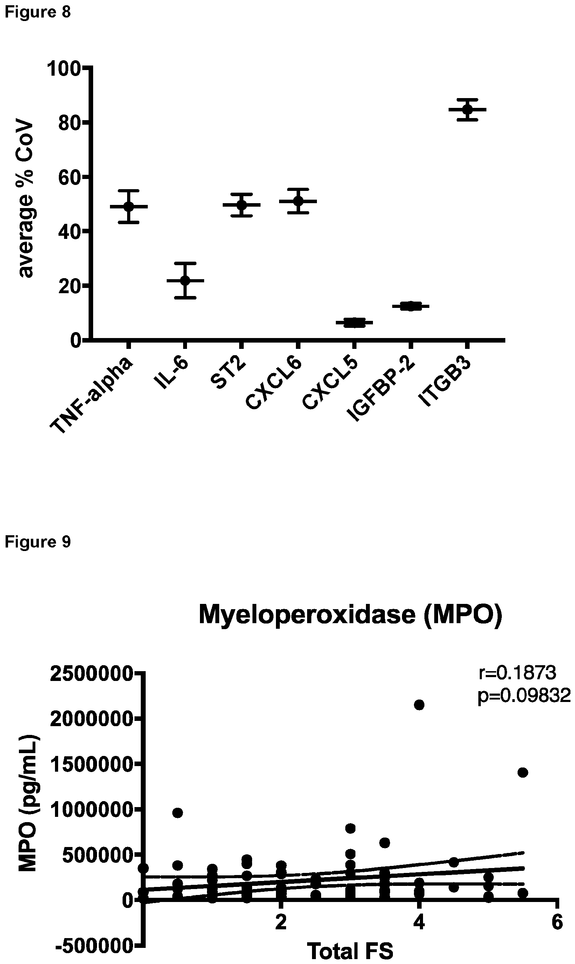

Figures 7 and 8 show the results of technical validation, analyzing percent coefficient of variation (CoV) observed between plates in healthy controls (Figure 7) and between subjects (Figure 8). These figures show the level of sample to sample variation.

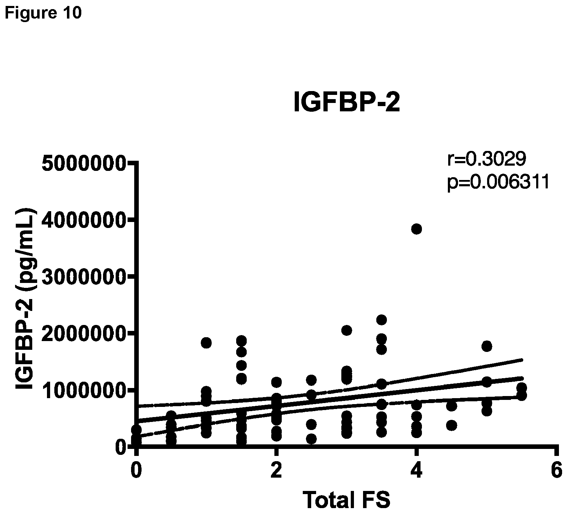

Figure 9 shows serum myeloperoxidase (MPO) levels as a function of Fazekas scores (FS), which weakly correlated. As shown in Figure 10, IGFPB2 serum levels were significantly correlated with FS scores.

These results confirm that serum markers, such as CXCL5/6 and IGFBP2 can be used to detect silent stroke. These assays can enable early treatment to reduce the risk of stroke and other deleterious consequences of endothelial disease.

Throughout this application various publications are referenced. The disclosures of these publications in their entireties are hereby incorporated by reference into this application in order to describe more fully the state of the art to which this invention pertains.

Those skilled in the art will appreciate that the conceptions and specific embodiments disclosed in the foregoing description may be readily utilized as a basis for modifying or designing other embodiments for carrying out the same purposes of the present invention. Those skilled in the art will also appreciate that such equivalent embodiments do not depart from the spirit and scope of the invention as set forth in the appended claims.

What is disclosed is:
1. A method of monitoring cerebrovascular status in a subject, the method comprising:
   (a) contacting a sample obtained from the subject with reagents that specifically bind to at least one marker selected from Table 1 or Table 2;
   (b) measuring the level of binding to the marker;
   (c) assigning a status score that reflects the measured amount of marker relative to a normal control;
   (d) referring the subject for treatment of metabolic syndrome and/or stroke if the status score is significantly greater than 1.
2. The method of statement 1, wherein the marker is C-X-C motif chemokine 5 (CXCL5) or C-X-C motif chemokine 6 (CXCL6).
3. The method of statement 1, wherein the marker is a marker of Table 1.
4. The method of statement 1, wherein the measuring of step (b) is performed for at least three of the markers of Table 1 and/or Table 2.
5. The method of any one of statements 1-4, further comprising measuring a marker of Table 3.
6. The method of any one of statements 1-5, wherein the sample is a CSF sample, a urine sample, a blood sample, or other bodily fluid.
7. A method of diagnosing silent stroke in a subject, the method comprising:
   (a) contacting a sample obtained from the subject with reagents that specifically bind to at least one marker selected from Table 1 or Table 2;
   (b) measuring the level of binding to the marker;
   (c) assigning a status score that reflects the measured amount of marker relative to a normal control;
   (d) referring the subject for treatment of metabolic syndrome and/or stroke if the status score is significantly greater than 1.
8. The method of any one of statements 1-7, further comprising treating the subject for metabolic syndrome and/or stroke.
9. The method of any one of statements 1-8, wherein the treatment is aspirin therapy, blood pressure therapy, body weight management, and/or a program of diet and exercise.
10. A method of monitoring silent stroke in a subject, the method comprising:
   (a) contacting a sample obtained from the subject with reagents that specifically bind to at least one marker selected from Table 1 or Table 2;
   (b) measuring the level of binding to the marker;
   (c) assigning a status score that reflects the measured amount of marker relative to a normal control;
   (d) treating the subject for metabolic syndrome and/or stroke if the status score is significantly greater than 1;
   (e) repeating steps (a) to (c); and
   (f) adjusting the treatment when the status score is not trending toward 1.
11. A method of treating silent stroke and/or metabolic syndrome in a subject in need thereof, the method comprising:
   (a) measuring a level of at least one marker selected from Table 1;
   (b) administering to the subject aspirin therapy, blood pressure therapy, blood sugar management, cholesterol management, body weight management, and/or a program of diet and exercise when the level of the marker is elevated.
12. The method of statement 11, wherein the at least one marker comprises C-X-C motif chemokine 5 (CXCL5) and/or C-X-C motif chemokine 6 (CXCL6).
13. The method of statement 11, wherein the at least one marker comprises IGFBP2.
14. The method of statement 11, wherein the measuring of step (a) is performed for at least two of markers of Table 1.
15. The method of statement 11, wherein the measuring of step (a) is performed for at least three of the markers of Table 1 and/or Table 2.
16. The method of any one of statements 11-15, further comprising measuring a marker of Table 3.
17. The method of any one of statements 11-16, wherein the sample is a CSF sample, a urine sample, a blood sample, or other bodily fluid.
18. The method of any of statements 1-10, wherein the reagent is an antibody or a nucleic acid probe.
19. The method of any of the preceding statements, wherein the measuring comprises immunoassay.
20. A kit comprising reagents that specifically bind each of CXCL5, CXCL6, IGFBP2, and optionally, ITGB3.

## Claims

1. A method of predicting, treating, and/or monitoring silent stroke in a subject in need thereof, the method comprising:
(a1) contacting a sample that has been obtained from the subject with reagents that specifically bind to at least two markers selected from the group consisting of: growth/differentiation factor 15 (GDF-15), interleukin-18 (IL-18), Insulin-like growth factor-binding protein 2 (IGFBP2), Integrin beta 3 (ITBG3), C-X-C motif chemokine 5 (CXCL5), C-X-C motif chemokine 6 (CXCL6), Chemokine (C-C motif) ligand 2 (MCP-1), fibroblast growth factor-23 (FGF-23), Fibrinogen, BDNF, ST2, SRAGE, myeloperoxidase (MPO), and LpPLA2; or
(a2) contacting a sample that has been obtained from the subject with a reagent that specifically binds to at least one marker selected from the group consisting of: growth/differentiation factor 15 (GDF-15), Insulin-like growth factor-binding protein 2 (IGFBP2), Integrin beta 3 (ITBG3), C-X-C motif chemokine 5 (CXCL5), C-X-C motif chemokine 6 (CXCL6), Chemokine (C-C motif) ligand 2 (MCP-1) and fibroblast growth factor-23 (FGF-23); or
(a3) contacting a sample that has been obtained from the subject with reagents that specifically bind to at least two markers selected from the group consisting of: growth/differentiation factor 15 (GDF-15), Insulin-like growth factor-binding protein 2 (IGFBP2), Integrin beta 3 (ITBG3), C-X-C motif chemokine 5 (CXCL5), C-X-C motif chemokine 6 (CXCL6), Chemokine (C-C motif) ligand 2 (MCP-1), fibroblast growth factor-23 (FGF-23), factor-alpha (TNFa) and Interleukin-6 (IL-6); and
(b) measuring a level of binding to the markers;
(c) assigning a status score that reflects the measured amount of markers relative to a normal control;
(d) referring the subject for treatment or monitoring if the status score is greater than 1.

2. The method of claim 1, wherein the measuring comprises performing an immunoassay.

3. The method of claim 1 or 2, wherein the sample is a serum sample, cerebrospinal fluid (CSF) sample, a urine sample, or a blood sample.

4. The method of any one of claims 1-3, wherein the reagents are or the reagent is an antibody.

5. The method of any one of claims 1-4, wherein the reagents are or the reagent is a nucleic acid probe.

6. The method of any one of claims 1-5 wherein the reagents specifically bind to GDF-15 and at least one marker selected from the group consisting of: CXCL5, CXCL6, IGFBP2, ITGB3, FGF-23, MCP-1, IL-6 and TNFa.

7. The method of any one of claims 1-5 wherein the reagents specifically bind to GDF-15 and IL-18.
